# FASCICULE DE BREVET EUROPEEN

(11) **EP 2 579 782 B1**
(45) Date de publication et mention de la délivrance du brevet: **05.09.2018**
(21) Numéro de dépôt: 11724236.2
(22) Date de dépôt: 09.06.2011
(51) Int. Cl.: A61B 10/02

(54) **OUTIL DE PRÉLÈVEMENT D'UN ÉCHANTILLON DE TISSU ANIMAL**
WERKZEUG ZUR ENTNAHME EIENR PROBE AUS TIERGEWEBE
TOOL FOR COLLECTING A SAMPLE OF ANIMAL TISSUE

(30) Priorité: 09.06.2010 FR 1054563
(43) Date de publication de la demande: 17.04.2013
(73) Titulaire: Allflex Europe, 35500 Vitré (FR)
(72) Inventeur: DESTOUMIEUX, Jean-Jacques, F-81380 Lescure-d'Albigeois (FR); TEYCHENE, Bruno, F-81430 Mouzieys-Teulet (FR)
(74) Mandataire: Vidon Brevets & Stratégie
(86) Numéro de dépôt international: PCT/EP2011/059636
(87) Numéro de publication internationale: WO 2011/154510

(56) Documents cités:
- EP-A1- 1 759 638
- WO-A1-01/89388
- WO-A1-96/26675
- WO-A1-2005/110602
- WO-A1-2008/003693
- WO-A1-2010/066475
- WO-A2-2007/087355
- US-A1- 2007 142 743
- US-B1- 6 196 978
- US-B1- 6 659 338

## Description

### 1. Domaine de l'invention

Le domaine de l'invention est celui du contrôle et/ou de l'identification des animaux.

Plus précisément, l'invention concerne le prélèvement de tissu d'un animal, permettant notamment de conserver des cellules portant des caractéristiques biologiques ou biochimiques de l'animal, par exemple pour identifier ultérieurement l'animal ou détecter des maladies de l'animal.

Encore plus précisément, l'invention concerne l'outil permettant de réaliser un tel prélèvement.

L'invention permet notamment le prélèvement de tissu sur des bovins, des ovins, des porcins, des caprins, des volatiles, des poissons ou plus généralement sur toute espèce animale, pouvant être effectué avec ou sans pose simultanée d'une marque d'identification.

### 2. Art antérieur

Afin d'améliorer le suivi du cheptel, améliorer la productivité (en éliminant les animaux malades, ou en recherchant des caractéristiques génétiques singulières par exemple), et garantir l'origine des animaux destinés notamment à la consommation (par exemple en détectant des maladies), il est de plus en plus souvent procédé à un ou plusieurs prélèvements de tissu des animaux concernés.

Un tel prélèvement peut être effectué directement sur l'animal, lors de la pose d'une marque d'identification de l'animal (à la naissance par exemple), ou plus tard. D'autres prélèvements peuvent également être effectués tout au long de l'existence de l'animal, par exemple pour détecter des maladies ou certifier l'identité de l'animal, par comparaison des séquences ADN. Une fois collecté, l'échantillon de tissu de l'animal peut donc être stocké et/ou transmis à un laboratoire pour analyse.

Les techniques de prélèvement actuellement utilisées permettent de prélever un échantillon de tissu de l'animal lors de la pose d'une marque d'identification, qui peut être visuelle ou électronique.

Malheureusement, ces techniques de prélèvement demandent une adaptation des marques existantes, tant au niveau de leur structure que des matériaux utilisés. Ces contraintes peuvent engendrer des problèmes de comportement des marques, comme une mauvaise tenue de la marque ou un vieillissement prématuré.

Des techniques de prélèvement de tissu mises en oeuvre indépendamment de la pose d'une marque d'identification sont également connues, par exemple du document US 6 659 339.

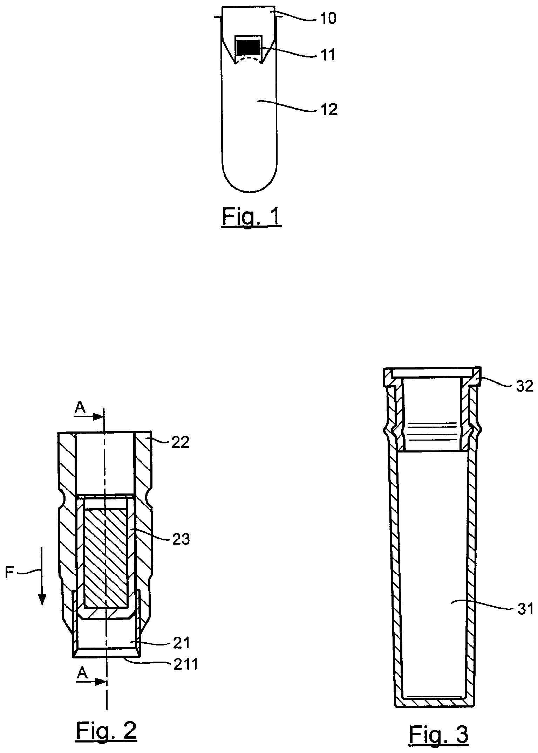

Comme illustré en figure 1, ces techniques reposent par exemple sur l'utilisation d'un emporte-pièce formant ou comprenant un élément de coupe 10, destiné à découper un échantillon 11 de tissu animal et à le collecter dans un logement. Pour ce faire, l'élément de coupe 10 présente une arête coupante de forme généralement circulaire, à contact continu ou en dents de scie.

L'emporte-pièce 10, fixé à une première mâchoire d'un outil de prélèvement, une pince par exemple, vient découper la peau de l'animal, et s'insérer au moins partiellement dans le microtube 12, fixé à une deuxième mâchoire de l'outil, lors de l'actionnement de l'outil. L'emporte-pièce 10 présente classiquement un diamètre légèrement inférieur à celui du microtube 12, de façon à servir de bouchon au microtube. Le logement recevant l'échantillon 11 est donc ouvert vers l'intérieur du microtube, mais fermé vers l'extérieur du microtube, de façon à éviter une contamination de l'échantillon.

Grâce à ces techniques de prélèvement, l'échantillon 11 est directement inséré dans le microtube 12, limitant ainsi le risque de contamination de l'échantillon.

Malheureusement, un inconvénient de cette technique est que des poils de l'animal restent souvent coincés entre les parois du microtube et l'emporte-pièce servant de bouchon, engendrant un problème d'étanchéité du réceptacle. La conservation de l'échantillon n'est donc pas assurée.

De plus, une fois l'emporte-pièce inséré dans le microtube, il n'est plus possible d'ajouter de produit dans le microtube, comme un agent conservateur ou un réactif, sans avoir à retirer ou découper l'emporte-pièce.

Encore un inconvénient de cette technique est que l'emporte-pièce doit être manipulé pour extraire l'échantillon de tissu collecté dans le logement, cette dernière opération pouvant se révéler malaisée et/ou complexe, et présenter un risque de coupure pour l'utilisateur.

D'autres techniques ont également été proposées pour le prélèvement de tissus.

Ainsi, le document WO 2005/110302 concerne un outil de type seringue permettant de prélever un échantillon de tissu animal, par exemple un échantillon de cerveau, pour détecter notamment si l'animal est atteint d'encéphalopathie spongiforme bovine.

Le document US2007/142743 concerne une technique de biopsie, mettant en oeuvre une aiguille formée d'un tube intérieur et d'un tube extérieur, mobiles l'un par rapport à l'autre.

Le document EP 1 759 638 concerne également une technique de biopsie, mettant en oeuvre une aiguille de biopsie comprenant une canule de biopsie, une canule en forme de cuillère et un stylet, pouvant coulisser les uns par rapport aux autres.

Le document WO01/89388 propose une sonde pour le prélèvement de tissus, comprenant un corps creux présentant une forme allongée et un élément de coupe conducteur. Le corps de la sonde est formée de deux parties qui peuvent coulisser l'une par rapport à l'autre, et définir un espace entre elles dans lequel la matière organique peut s'étendre.

Le document WO 2007/087355 propose un dispositif permettant de prélever des échantillons à un endroit du corps et de les réimplanter à un autre endroit du corps du patient. Par exemple, un tel dispositif comprend un tube creux, un stylet et des moyens d'arrêt. Le tube creux présente une arête coupante.

Le document WO 2010/066475, qui relève de l'article 54(3) CBE, ou encore le document FR 08-58453, proposent un outil de prélèvement d'un échantillon de tissu animal, destiné à coopérer avec des moyens de prélèvement comprenant au moins un élément de coupe apte à découper ledit échantillon et un élément poussoir mobile par rapport audit élément de coupe, apte à pousser ledit échantillon dans des moyens de stockage après découpe, lesdits moyens de stockage comprenant un tube d'échantillonnage et une tête de tube. Cet outil comprend des moyens d'entraînement dudit élément de coupe, permettant de mouvoir ledit élément de coupe selon un axe de translation, en direction dudit tissu, et des moyens d'entraînement dudit élément poussoir, permettant de mouvoir ledit élément poussoir selon ledit axe de translation en direction dudit tissu, jusqu'à insertion d'au moins une portion dudit élément poussoir dans ladite tête de tube. Ledit outil comprend en outre un ressort de longueur calibrée permettant d'accoupler lesdits moyens d'entraînement de l'élément de coupe et lesdits moyens d'entraînement de l'élément poussoir. Il existe toutefois un besoin pour une nouvelle technique de prélèvement de tissu d'un animal ne présentant pas l'ensemble des inconvénients de l'art antérieur.

### 3. Exposé de l'invention

L'invention propose une solution nouvelle qui ne présente pas l'ensemble de ces inconvénients de l'art antérieur, sous la forme d'un outil de prélèvement d'un échantillon de tissu animal selon la revendication 1. Différents modes de réalisation préférentiels sont définis dans les revendications dépendantes. L'invention propose ainsi un nouvel outil pour le prélèvement de tissu animal, permettant d'actionner simultanément des moyens d'entraînement de l'élément de coupe et des moyens d'entraînement de l'élément poussoir dans un premier temps, puis uniquement les moyens d'entraînement de l'élément poussoir dans un deuxième temps, à partir d'une seule action sur l'outil.

En d'autres termes, les opérations de découpe du tissu, extraction du tissu et fermeture du tube sont réalisées en une seule action, soit un seul mouvement pour l'utilisateur (actionnement d'un levier, déclenchement d'une gâchette, etc).

L'outil proposé peut donc être vu comme un mécanisme à double axe (ou double pointeau), un axe (ou pointeau) primaire transmettant un mouvement de translation à l'élément de coupe pour découper l'échantillon de tissu, et un axe (ou pointeau) secondaire transmettant un mouvement de translation à l'élément poussoir selon une même direction pour extraire le tissu découpé, ces deux axes étant solidarisés jusqu'à la découpe du tissu, puis désolidarisés ensuite. La trajectoire de l'élément de coupe et de l'élément poussoir au travers des tissus animal (oreille par exemple) est donc rectiligne.

L'invention permet ainsi un prélèvement optimisé, particulièrement simple et rapide pour l'utilisateur, qui n'a pas à réaliser lui-même plusieurs actions pour perforer le tissu animal, pousser l'échantillon dans des moyens de stockage, fermer les moyens de stockage, etc, toutes ces opérations étant réalisées à partir d'une seule action sur l'outil (par exemple une action manuelle, électrique, pneumatique ou autre sur les poignées de l'outil).

De plus, on évite une contamination potentielle de l'échantillon, puisque l'utilisateur n'a pas à agir directement sur l'échantillon. Par ailleurs, l'échantillon étant découpé par l'élément de coupe, puis automatiquement poussé dans les moyens de stockage par un élément poussoir, aucune portion de l'outil ou aucun élément extérieur n'est en contact direct avec le tissu prélevé.

Selon une caractéristique particulière de l'invention, l'outil de prélèvement comprend au moins une poignée articulée formant levier, mobile sur une course prédéterminée comprenant une première portion et une deuxième portion.

Un tel levier comprend des moyens d'action sur les moyens d'accouplement permettant le passage de la position d'accouplement sur la première portion à la position de désaccouplement sur la deuxième portion.

Ainsi, lorsque l'utilisateur exerce une pression sur le levier, celui-ci passe :
- d'une position initiale à une position intermédiaire, correspondant à une première portion de la course du levier au cours de laquelle les moyens d'entraînement de l'élément de coupe et les moyens d'entraînement de l'élément poussoir sont accouplés ; puis
- de la position intermédiaire à une position finale, correspondant à une deuxième portion de la course du levier, au cours de laquelle les moyens d'entraînement de l'élément de coupe et les moyens d'entraînement de l'élément poussoir sont désaccouplés.
Selon une caractéristique essentielle de l'invention, les moyens d'accouplement mettent en oeuvre au moins un élément de couplage mobile, apte à coopérer avec un logement ménagé à cet effet dans les moyens d'entraînement de l'élément de coupe, le ou les éléments de couplage étant maintenus dans le logement dans la position d'accouplement et libérés dans la position de dés accouplement.

On note que plusieurs éléments de couplage peuvent être prévus (par exemple un, deux ou trois).

Ce mode de réalisation permet de s'affranchir de l'utilisation de ressorts précontraints, prévus pour se comprimer à partir d'un certain effort, tel celui décrit dans WO 2010/066475. Ainsi, dans la position de désaccouplement, il n'est pas nécessaire de cumuler les efforts de découpe et les efforts pour fermer les moyens de stockage.

De plus, selon ce mode de réalisation, les moyens d'accouplement délient les moyens d'entraînement de l'élément de coupe des moyens d'entraînement de l'élément poussoir en fonction de la course du levier, et non des différents efforts entrant en jeu : effort résistant pour traverser les tissus animal (qui diffèrent selon la zone où le prélèvement est effectué (du fait d'une variation d'épaisseur des tissus notamment), selon la race ou la catégorie de l'animal, selon l'orientation de l'outil par rapport aux tissus à prélever, etc), effort pour fermer les moyens de stockage, effort exercé sur le levier, etc. Ceci permet une meilleure répétabilité de l'opération, puisque la course du levier est toujours identique alors que l'effort exercé sur le levier peut être variable.

En particulier, selon ce mode de réalisation, les moyens d'entraînement de l'élément poussoir comprennent au moins une gorge, et le logement est en regard de la gorge dans la position d'accouplement.

De cette façon, dans la position d'accouplement, la gorge reçoit également l'élément d'accouplement prévu dans le logement correspondant.

Par exemple, l'élément de couplage est une bille, un cylindre, etc.

Selon un aspect particulier, le logement est une lumière traversante et la bille est maintenue dans le logement par l'intermédiaire d'une pièce coulisseau, conformée pour définir des moyens de maintien maintenant la bille dans la gorge dans la position d'accouplement, et des moyens de libération libérant la bille de la gorge dans la position de dés accouplement.

Par exemple, la pièce coulisseau présente une forme de révolution autour de l'axe de translation, définissant un manchon comprenant au moins deux sections de diamètres distincts, les moyens de maintien correspondant à la section de diamètre inférieur et les moyens de libération à la section de diamètre supérieur.

La pièce coulisseau peut être est reliée aux moyens d'entraînement de l'élément poussoir par un ressort, dit ressort coulisseau, le ressort coulisseau tendant à écarter la pièce coulisseau des moyens d'entraînement de l'élément poussoir. Par exemple, le ressort coulisseau est dans un état de repos dans la position d'accouplement et dans un état de compression dans la position de désaccouplement.

Selon une autre caractéristique de l'invention, l'outil de prélèvement comprend des moyens de verrouillage des moyens de stockage sur une branche de l'outil.

Par exemple, ces moyens de verrouillage sont de type bague de verrouillage, élément translatant, etc.

Ils permettent de maintenir les moyens de stockage, par exemple un tube d'échantillonnage, de manière sûre, lors du prélèvement. Une fois le prélèvement effectué, l'utilisateur peut simplement déverrouiller ces moyens pour avoir accès au tube contenant l'échantillon. De cette façon, l'utilisateur n'a pas à manipuler directement l'échantillon.

Selon une autre caractéristique, l'outil de prélèvement comprend des moyens d'éjection de l'élément de coupe.

Par exemple, après découpe des tissus, l'élément de coupe usagé est ramené en position initiale et éjecté de l'outil par une action volontaire de l'utilisateur, comme une action en sens inverse du levier par exemple, sans contact direct avec l'utilisateur, afin de jeter l'élément de coupe dans une zone adéquate (poubelle, bac de récupération, etc).

De cette façon, l'utilisateur n'a pas à manipuler l'élément de coupe souillé, ce qui évite le risque de blessure et de contamination.

L'outil de prélèvement est donc conçu de manière à ce qu'à aucun moment l'utilisateur ne soit en contact direct avec l'élément de coupe souillé et l'échantillon prélevé.

Selon un aspect particulier de l'invention, l'outil de prélèvement comprend des moyens de retrait automatique de l'élément de coupe.

De tels moyens permettent de libérer rapidement l'oreille de l'animal, et évitent les risques de coupure de l'utilisateur ou de l'animal avec un élément de coupe souillé, en protégeant l'arête coupante de l'élément de coupe.

### 4. Liste des figures

D'autres caractéristiques et avantages de l'invention apparaîtront plus clairement à la lecture de la description suivante d'un mode de réalisation particulier, donné à titre de simple exemple illustratif et non limitatif, et des dessins annexés, parmi lesquels :
- la figure 1 illustre un dispositif de prélèvement d'un échantillon de tissu selon l'art antérieur ;
- les figures 2 et 3 présentent respectivement une vue en coupe des moyens de prélèvement et des moyens de stockage pouvant être mis en oeuvre par un outil de prélèvement selon un mode de réalisation de l'invention ;
- la figure 4 illustre un outil de prélèvement selon un mode de réalisation de l'invention, en position initiale ;
- les figures 5A et 5B illustrent plus précisément les différents éléments de l'outil de prélèvement selon la figure 4 au cours du prélèvement ;
- les figures 6 et 7 présentent un exemple de mécanisme d'éjection de l'élément de coupe de l'outil de prélèvement de la figure 4 ;
- les figures 8A et 8B illustrent respectivement l'outil de prélèvement de la figure 4 et les moyens de stockage après l'opération de prélèvement ;
- les figures 9A et 9B proposent un exemple de réalisation des moyens d'accouplement selon l'invention ;
- la figure 10 présente un deuxième exemple de réalisation des moyens d'accouplement ne rentrant pas dans le cadre de la présente invention ;
- les figures 11A et 11B illustrent un troisième exemple de réalisation des moyens d'accouplement ne rentrant pas dans le cadre de la présente invention ;
- les figures 12A à 12H illustrent un autre exemple de mise en oeuvre d'un outil de prélèvement ne rentrant pas dans le cadre de la présente invention ;
- les figures 13A à 15 présentent différentes techniques permettant un retrait automatique de l'élément de coupe après découpage des tissus.

### 5. Description d'un mode de réalisation de l'invention

### 5.1 Principe général

Le principe général de l'invention repose sur un outil de prélèvement spécifique, fonctionnant en deux temps, permettant l'utilisation de moyens de prélèvement comprenant un élément de coupe et un élément poussoir pour prélever un échantillon de tissu animal, tels que décrits par exemple dans la demande de brevet français déposée sous le numéro FR-08 58453.

Plus précisément, un tel outil de prélèvement permet de déplacer simultanément l'élément de coupe et l'élément poussoir dans un premier temps, puis de déplacer uniquement l'élément poussoir dans un deuxième temps.

L'outil selon l'invention comprend ainsi des moyens d'entraînement de l'élément de coupe et des moyens d'entraînement de l'élément poussoir, permettant de mouvoir l'élément de coupe et l'élément poussoir en direction du tissu à prélever, et des moyens d'accouplement réversible des moyens d'entraînement de l'élément de coupe et des moyens d'entraînement de l'élément poussoir.

Ces moyens d'accouplement permettent de définir deux positions :
∘ une position d'accouplement permettant de lier les mouvements des éléments de coupe et poussoir, et donc de déplacer simultanément ces deux éléments, et
∘ une position de désaccouplement permettant de dissocier les mouvements des éléments de coupe et poussoir, et donc de déplacer séparément l'élément poussoir.

### 5.2 Description d'un mode de réalisation particulier

On décrit ci-après un mode de réalisation particulier, selon lequel l'outil de prélèvement selon l'invention est utilisé pour réaliser un prélèvement en utilisant les moyens de prélèvement et de stockage tels que décrits dans la demande de brevet français FR-08 58453.

### A) Moyens de prélèvement

On rappelle ci-après les principales caractéristiques des moyens de prélèvement décrits dans la demande de brevet français FR-08 58453.

Comme illustré en figure 2, ces moyens de prélèvement comprennent d'une part un élément de coupe 21, et d'autre part un élément poussoir 23. L'élément de coupe 21 peut notamment être solidarisé à un support 22. Ce support 22, qui peut être réalisé en matière plastique, est destiné à être monté à l'extrémité d'un pointeau solidarisé à une des branches d'un outil de prélèvement, de façon amovible. Il se présente sous la forme d'une surface de révolution, de même axe que l'élément de coupe 21 et l'élément poussoir 23. Il comprend notamment une collerette 221, sur laquelle peut venir se fixer le pointeau pour entraîner l'élément de coupe.

Selon une variante, l'élément de coupe 21 et le support 22 sont formés d'un seul tenant, par exemple en plastique ou en métal. On considère alors que l'élément de coupe 21 et le support 22 forment une seule pièce, qui est « monobloc ».

On considère par exemple que l'élément de coupe 21 présente une forme de révolution cylindrique. L'arête coupante 211 présente alors une forme circulaire.

Le cylindre formant élément de coupe 21 est ouvert à ses deux extrémités, afin de laisser passer l'élément poussoir 23, de sorte que ce dernier puisse pousser l'échantillon hors de l'élément de coupe 21 et l'accompagner dans des moyens de stockage, selon la direction illustrée par la flèche F.

Selon l'exemple de réalisation illustré, l'élément poussoir 23 présente également une forme de révolution cylindrique. Toutefois, le diamètre de l'élément poussoir 23 doit être inférieur à celui de l'élément de coupe 21 pour que l'élément poussoir 23 puisse glisser à l'intérieur du cylindre formé par l'élément de coupe 21.

L'élément poussoir 23 est mobile en translation suivant l'axe A-A à l'intérieur de l'élément de coupe 21, selon la direction illustrée par la flèche F.

### B) Moyens de stockage

On rappelle également les principales caractéristiques des moyens de stockage décrits dans la demande de brevet français FR-08 58453.

Comme illustré en figure 3, ces moyens de stockage comprennent d'une part un tube d'échantillonnage 31 comprenant au moins une ouverture, et d'autre part une tête de tube 32, solidarisée à l'entrée du tube d'échantillonnage, par exemple par clippage ou emboîtement. Elle peut être réalisée en matière flexible, notamment en caoutchouc, pour faciliter son insertion dans le col du tube.

Plus précisément, la tête de tube 32 se présente sous la forme d'un capuchon percé d'une ouverture centrale, de diamètre suffisant pour permettre l'insertion d'au moins une portion de l'élément poussoir 23. L'utilisation d'une matière flexible pour ce capuchon permet aussi de faciliter l'introduction de l'élément poussoir 23 dans le tube 31. L'élément poussoir 23 ferme alors de façon étanche ou quasi-étanche le tube 31.

La tête de tube 32 présente également une collerette, prenant appui sur le rebord du tube d'échantillon 31, définissant une surface de butée (ou billot) sur laquelle l'arête coupante de l'élément de coupe 21 peut prendre appui lors du prélèvement, afin de découper plus facilement l'échantillon de tissu.

### C) Outil de prélèvement

On décrit ci-après plusieurs exemples de l'outil de prélèvement, parfois également appelé outil de pose ou pince, pouvant être utilisé pour prélever un échantillon de tissu selon l'invention.

### i) Fonctionnement général

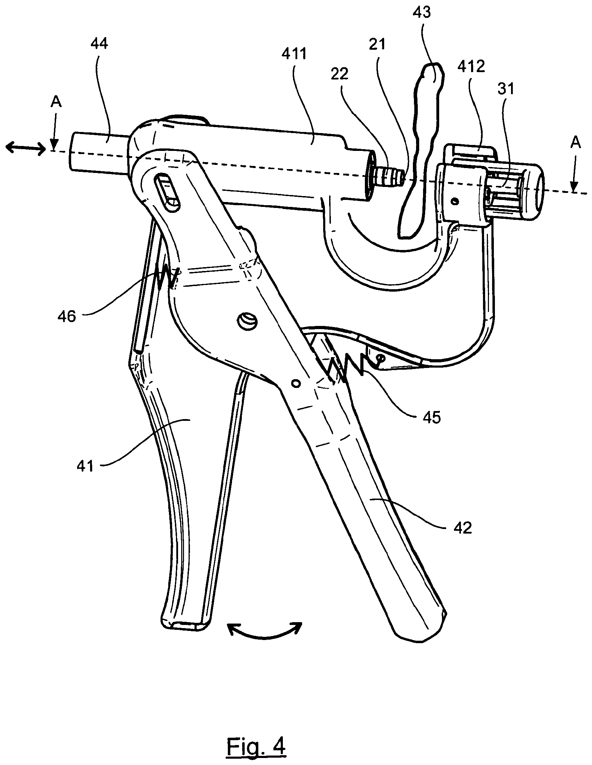

Comme illustré en figure 4, un tel outil comprend généralement une partie fixe encore appelée corps 41, définissant notamment une première poignée, et une partie articulée encore appelée levier 42, définissant une deuxième poignée.

Le corps 41 de l'outil définit également deux branches entre lesquelles on vient positionner le tissu à prélever, par exemple l'oreille 43 de l'animal. Ainsi, la première branche 411 est destinée à coopérer avec des moyens de prélèvement tels qu'illustrés en figure 2 par exemple, et la deuxième branche 412 est destinée à coopérer avec des moyens de stockage tels qu'illustrés en figure 3 par exemple.

Plus précisément, la deuxième branche 412 comprend des moyens de verrouillage du tube d'échantillonnage, comme une bague de verrouillage par exemple.

La première branche 411 comprend quant à elle des moyens d'entraînement 44 des moyens de prélèvement, permettant de mouvoir l'élément de coupe 21 (et/ou son support 22) et l'élément poussoir 23 en translation en direction du tissu à prélever, selon l'axe de translation A-A. Ces moyens d'entraînement sont activés lorsque l'utilisateur de l'outil exerce une force sur le levier 42.

Ainsi, le levier 42, actionnable manuellement par exemple, agit sur les moyens d'entraînement 44, de façon à guider les moyens de prélèvement selon un mouvement de translation, pour qu'ils pénètrent la chair de l'animal et forcent l'échantillon prélevé dans les moyens de stockage. L'outil peut également être actionné au moyen d'une énergie électrique, pneumatique, ou autre. Les moyens de prélèvement et de stockage sont donc configurés pour coopérer avec un tel outil.

Plus précisément, les moyens d'entraînement 44 comprennent des moyens d'entraînement de l'élément de coupe 21 (et/ou de son support 22), des moyens d'entraînement de l'élément poussoir 23, et des moyens d'accouplement réversible de ces moyens d'entraînement. Ainsi, dans un premier temps, correspondant à une première portion de la course du levier 42 entre une position initiale et une position intermédiaire, les moyens d'entraînement de l'élément de coupe 21 et les moyens d'entraînement de l'élément poussoir 23 sont accouplés, dans une position d'accouplement permettant de lier les mouvements des éléments de coupe et poussoir. Dans un deuxième temps, correspondant à une deuxième portion de la course du levier 42 entre la position intermédiaire et une position finale, les moyens d'entraînement de l'élément de coupe 21 et les moyens d'entraînement de l'élément poussoir 23 sont désaccouplés, dans une position de désaccouplement permettant de dissocier les mouvements des éléments de coupe et poussoir.

La figure 4 illustre l'état initial de l'outil de pose, lorsque les moyens de prélèvement et de stockage sont montés sur l'outil, qui est prêt à être utilisé.

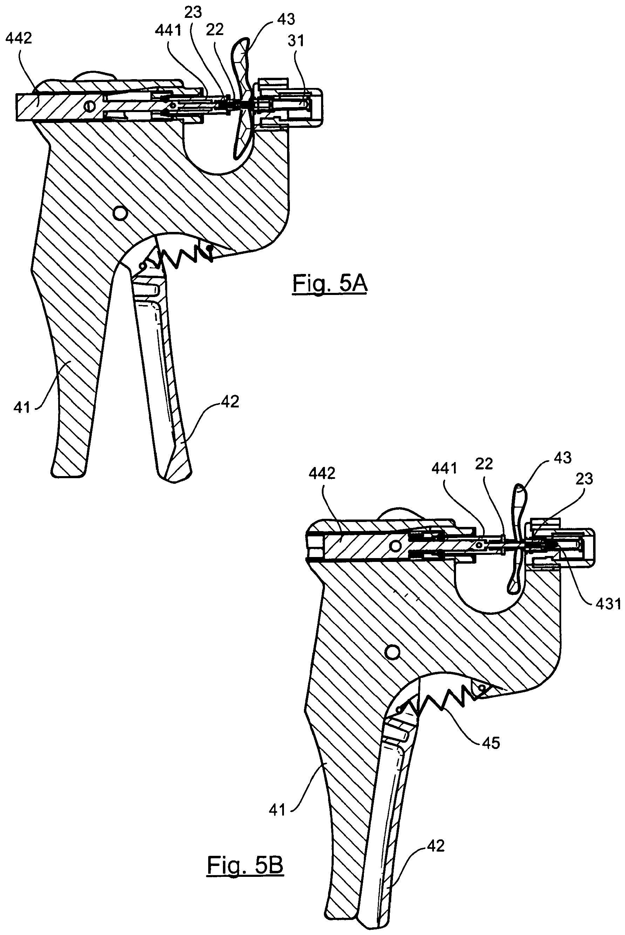

Les figures 5A et 5B illustrent plus précisément les différents éléments de l'outil de prélèvement au cours du prélèvement, et notamment les deux positions d'accouplement et de désaccouplement. On considère par exemple, que l'élément de coupe 21 est guidé en translation par des moyens d'entraînement 441, et que l'élément poussoir 23 est guidé en translation par des moyens d'entraînement 442, poussant l'élément poussoir 23 au travers de l'élément de coupe 21.

Comme illustré en figure 5A, les moyens d'entraînement 441 de l'élément de coupe et les moyens d'entraînement 442 de l'élément poussoir sont liés dans une position d'accouplement dans un premier temps, correspondant à la première portion de la course du levier 42. L'action sur le levier 42 entre sa position initiale et sa position intermédiaire, correspondant à la première portion de sa course, engendre alors une translation simultanée de l'élément de coupe 21 (ou de son support 22) et de l'élément poussoir 23, selon le même axe de translation.

Cette translation permet à l'élément de coupe de perforer l'oreille 43 selon une trajectoire rectiligne pour découper un échantillon de tissu, jusqu'à venir en butée sur la collerette de la tête de tube du tube d'échantillonnage 31.

Dans un deuxième temps correspondant à une deuxième portion de la course du levier 42, tel qu'illustré en figure 5B, les moyens d'entraînement 441 de l'élément de coupe et les moyens d'entraînement 442 de l'élément poussoir ne sont plus liés. Ainsi, dans cette position de désaccouplement, la poursuite de l'action sur le levier 42 sur cette deuxième portion engendre une translation de l'élément poussoir 23 uniquement. L'élément poussoir 23 est alors guidé en translation à travers l'élément de coupe 21 (bloqué en butée contre la collerette de la tête de tube), et pousse l'échantillon 431 découpé jusque dans le tube 31. L'élément poussoir 23 termine sa course en s'emboîtant dans la tête du tube, et obture ainsi hermétiquement ou quasi-hermétiquement le tube 31.

En d'autres termes, l'action de l'élément poussoir débute après que l'élément de coupe a assuré la découpe du tissu contre une « enclume » (collerette de la tête de tube du tube d'échantillonnage 31), garantissant ainsi une découpe nette et répétable de la première peau, du cartilage, et de la deuxième peau.

Le prélèvement étant réalisé de manière certaine, l'utilisateur peut alors relâcher le levier 42. Un ressort de rappel 45, ou tout autre moyen, peut alors ramener le levier dans sa position initiale, ainsi que les moyens d'entraînement 441 de l'élément de coupe et les moyens d'entraînement 442 de l'élément poussoir dans leur position initiale, c'est-à-dire dans leur position d'accouplement.

L'élément de coupe peut ainsi être ramené en position initiale. De cette façon, l'élément de coupe ne reste ni dans les moyens de stockage, ni dans l'animal, et peut présenter une forme quelconque particulièrement bien adaptée à la découpe de tissu animal, et notamment une arête extrêmement coupante.

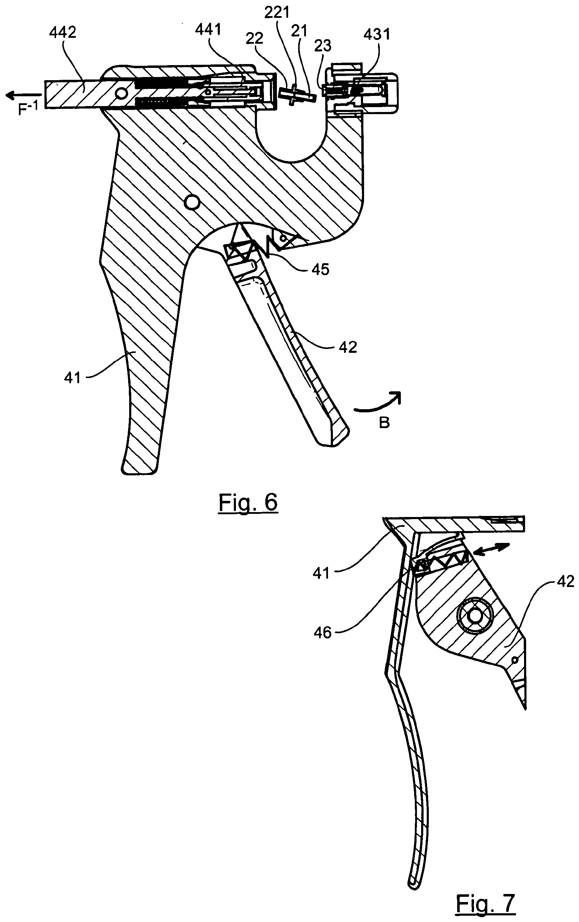

Selon un aspect particulier de l'invention, illustré en figure 6, l'outil de prélèvement prévoit des moyens d'éjection de l'élément de coupe 21 après utilisation. Par exemple, l'utilisateur peut appliquer un mouvement de rotation (ou de translation) inverse au levier 42, tendant à l'écarter de la première poignée 41 au lieu de l'en approcher (flèche B). Ce mouvement de rotation (ou de translation) inverse engendre une translation des moyens d'entraînement 441 de l'élément de coupe et des moyens d'entraînement 442 de l'élément poussoir dans une direction F⁻¹, opposée à la direction de prélèvement F. Les moyens d'entraînement 441 de l'élément de coupe entraînent alors l'élément de coupe 21 selon la direction F⁻¹, jusqu'à ce que la collerette 221 du support 22 de l'élément de coupe viennent en butée sur des moyens prévus à cet effet sur le corps de l'outil. Le support 22 et l'élément de coupe 21 se trouvent alors désolidarisés des moyens d'entraînement 441, et éjectés de l'outil de prélèvement, sans que l'utilisateur ait besoin de toucher l'élément de coupe souillé.

L'utilisateur peut ainsi éjecter l'élément de coupe souillé quand il le souhaite, dans un lieu adéquat, sans avoir à toucher cet élément, ce qui permet d'éviter les risques de coupure ou de contamination.

Préalablement à l'éjection, l'utilisateur peut bien entendu positionner un élément protecteur sur l'élément de coupe 21.

Selon cet aspect, on considère que la butée du levier en position initiale (ou position de repos) est élastique. Elle peut être réalisée par un système amortisseur ou par un ressort 46, comme illustré en figure 7, ramenant le levier 42 et tout le mécanisme en position initiale. Le mouvement du levier 42 par rapport au corps 41 dans le sens opposé au sens de prélèvement est donc permis.

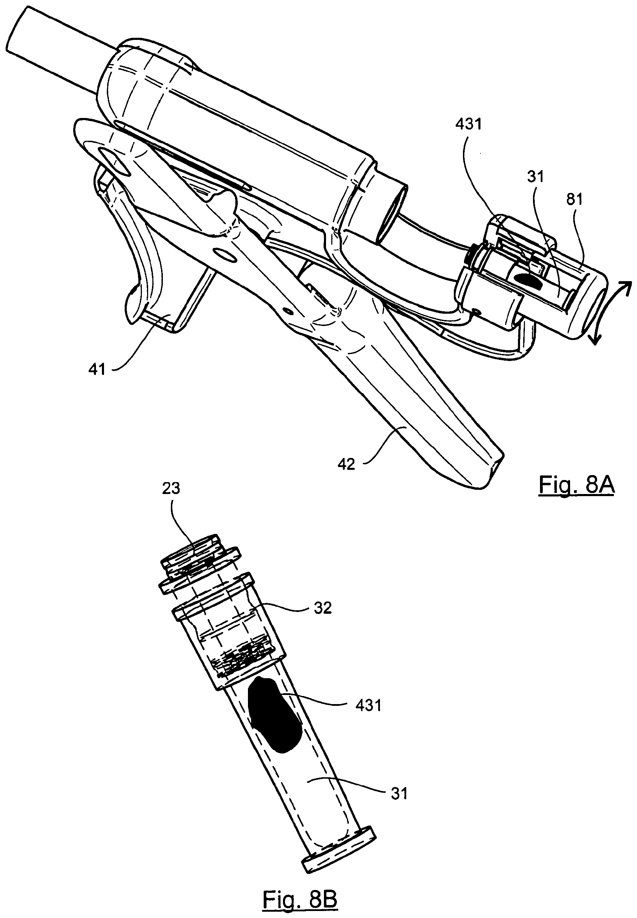

Finalement, l'utilisateur peut déverrouiller les moyens de verrouillage 81 du tube 31, par exemple en tournant une bague de verrouillage, ou en translatant un élément de verrouillage, afin de récupérer l'échantillon 431 conditionné dans le tube 31, comme illustré en figure 8A. Comme illustré en figure 8B, l'échantillon 431 est alors prêt pour être transmis à un laboratoire, dans son tube d'échantillonnage 31 obturé par l'élément poussoir 23.

### ii) Exemple de réalisation des moyens d'accouplement selon l'invention

On décrit ci-après un exemple de réalisation des moyens d'accouplement, permettant le passage d'une position d'accouplement des moyens d'entraînement 441 de l'élément de coupe et des moyens d'entraînement 442 à une position de désaccouplement.

Selon la présente invention, les moyens d'accouplement mettent en oeuvre au moins un élément de couplage mobile, apte à coopérer avec un logement ménagé à cet effet dans les moyens d'entraînement 441 de l'élément de coupe. Ces éléments de couplage (billes, cylindres, ou autre) sont maintenus dans le logement dans la position d'accouplement, et libérés dans la position de désaccouplement.

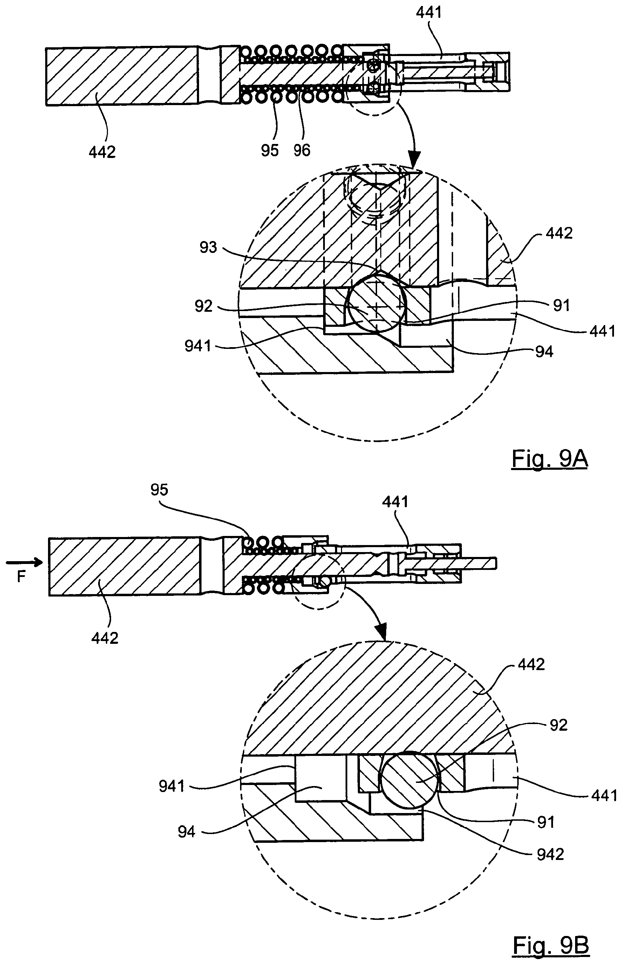

Plus précisément, les figures 9A et 9B illustrent les moyens d'entraînement 441 et 442 de l'élément de coupe et de l'élément poussoir, correspondant aux positions illustrées respectivement en figures 5A et 5B.

On considère selon ce premier exemple que les moyens d'entraînement 441 de l'élément de coupe comprennent un ou plusieurs logements 91 recevant chacun une bille 92. Les moyens d'entraînement 442 de l'élément poussoir comprennent quant à eux au moins une gorge 93, telle que chaque logement 91 est en regard de la gorge 93 dans la position d'accouplement.

Dans la position d'accouplement illustrée en figure 9A, le logement 91 est traversant (lumière) et la bille 92 est maintenue dans le logement 91 par l'intermédiaire d'une pièce coulisseau 94. Dans la position de désaccouplement illustrée en figure 9B, la bille 92 n'est plus maintenue par l'intermédiaire de la pièce coulisseau 94.

Par exemple, une telle pièce coulisseau 94 présente une forme de révolution autour de l'axe de translation, définissant un manchon comprenant au moins deux sections de diamètres distincts. La section 941 de plus petit diamètre permet de maintenir la bille 92 dans la gorge 93 dans la position d'accouplement. La section 942 de plus grand diamètre permet de libérer la bille 92 de la gorge 93 dans la position de désaccouplement.

La pièce coulisseau 94 est reliée aux moyens d'entraînement 442 de l'élément poussoir par un ressort, dit ressort coulisseau 95, proposé selon cet exemple dans un état de repos dans la position d'accouplement et dans un état de compression dans la position de désaccouplement.

Plus précisément, lors du prélèvement, les moyens d'entraînement 441 de l'élément de coupe et les moyens d'entraînement 442 de l'élément poussoir sont accouplés dans un premier temps, comme illustré en figure 9A, ce qui engendre la translation simultanée de l'élément de coupe et de l'élément poussoir.

L'élément de coupe perfore alors l'oreille de l'animal pour découper un échantillon de tissu. Juste avant que l'élément de coupe vienne en butée sur la collerette de la tête de tube du tube d'échantillonnage, la pièce coulisseau 94 se retrouve également en butée contre un élément de butée (tel qu'un épaulement) prévu à cet effet sur le corps de l'outil, au niveau de sa section 942 de diamètre supérieur. Comme l'utilisateur poursuit son action sur le levier 42 alors que la pièce coulisseau 94 se trouve en butée, les moyens d'entraînement 442 de l'élément poussoir continuent à pousser l'élément poussoir 23, et guident la ou les billes 92, qui étaient jusque-là maintenues par la section 941 de plus petit diamètre de la pièce coulisseau 94, vers la section 942 de plus grand diamètre. Les billes 92 sont alors libérées, et les moyens d'entraînement 441 de l'élément de coupe et les moyens d'entraînement 442 de l'élément poussoir se trouvent désaccouplés, comme illustré en figure 9B, et l'élément poussoir peut continuer son mouvement indépendamment de l'élément de coupe.

Au moment où les billes sont libérées, l'élément de coupe se trouve en butée sur la collerette de la tête de tube du tube d'échantillonnage.

On note que le ressort primaire 96, prévu entre les moyens d'entraînement 441 de l'élément de coupe et les moyens d'entraînement 442 de l'élément poussoir, permet de maintenir en pression l'élément de coupe contre la collerette de la tête de tube du tube d'échantillonnage, notamment lorsque le ressort coulisseau 95 se comprime.

### iii) Deuxième exemple illustratif de réalisation des moyens d'accouplement

On décrit ci-après un deuxième exemple de réalisation des moyens d'accouplement, mettant en oeuvre un système de type « dévêtisseur ».

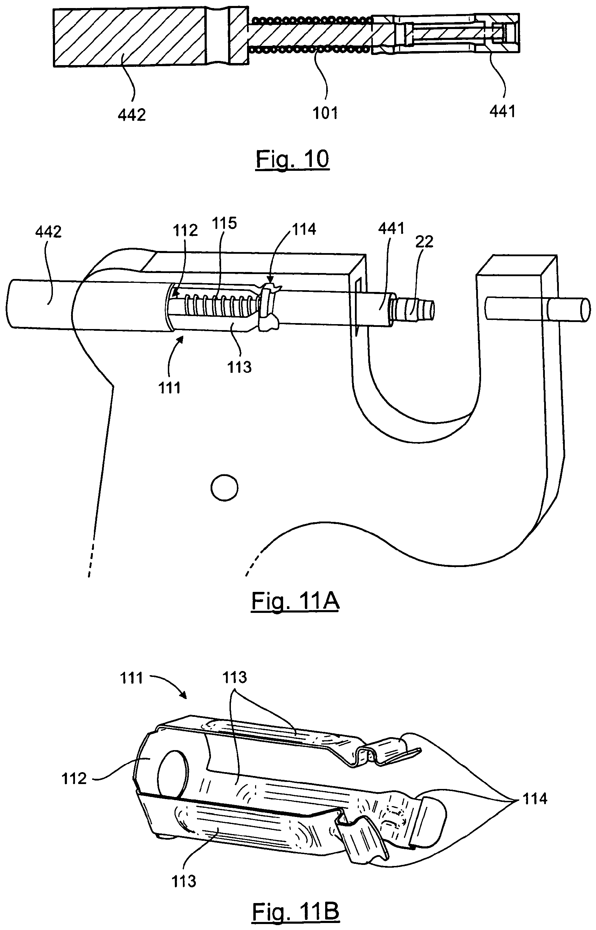

Selon cet exemple, illustré en figure 10, on prévoit un ressort de couplage 101, positionné entre les moyens d'entraînement 441 de l'élément de coupe et les moyens d'entraînement 442 de l'élément poussoir.

Plus précisément, un tel ressort de couplage est précontraint, et prévu pour se comprimer lorsqu'on lui applique une force supérieure à la force nécessaire à l'élément de coupe pour découper l'échantillon. On s'assure de cette manière que les mouvements de l'élément de coupe 21 et de l'élément poussoir 23 sont identiques (moyens d'entraînement 441 de l'élément de coupe et moyens d'entraînement 442 de l'élément poussoir dans une position d'accouplement), tant que l'élément de coupe n'a pas perforé le tissu animal.

En effet, tant que l'élément de coupe n'est pas en butée sur la collerette de la tête de tube du tube d'échantillonnage, le ressort de couplage 101 ne se comprime pas, car la force exercée sur l'élément de coupe est suffisante pour perforer le tissu animal, mais insuffisante pour comprimer le ressort de couplage 101.

Une fois que l'élément de coupe se trouve en butée sur la collerette de la tête de tube du tube d'échantillonnage, le ressort de couplage 101 se comprime si l'utilisateur poursuit son action sur le levier 42, la force exercée sur l'élément de coupe étant alors supérieure à celle nécessaire pour perforer le tissu animal.

Par exemple, si l'on considère que la force nécessaire à l'élément de coupe pour découper l'échantillon est de l'ordre de 60 Newton, le ressort de couplage 101 peut être précontraint à une force de l'ordre de 80 ou 100 N.

### iv) Troisième exemple illustratif de réalisation des moyens d'accouplement

On décrit ci-après un troisième exemple de réalisation des moyens d'accouplement, mettant en oeuvre un système de clippage avec au moins un élément au moins partiellement déformable.

Comme illustré en figures 11A et 11B, l'élément au moins partiellement déformable 111 comprend par exemple une base 112, destinée à venir prendre appui sur les moyens d'entraînement 442 de l'élément poussoir, prenant par exemple la forme d'un pointeau. Une pluralité de doigts, par exemple trois doigts 113, s'étendent à partir de cette base 112, sensiblement parallèlement à l'axe de translation des éléments de coupe et poussoir.

Chaque doigt 113 comprend un bossage ou décrochement 114, apte à coopérer avec un logement complémentaire prévu dans les moyens d'entraînement 441 de l'élément de coupe dans la position d'accouplement.

Plus précisément, les doigts 113 forment des lames ressort, permettant de maintenir les moyens d'entraînement 441 de l'élément de coupe dans la position d'accouplement.

Lors du prélèvement, les moyens d'entraînement 441 de l'élément de coupe et les moyens d'entraînement 442 de l'élément poussoir sont accouplés dans un premier temps, au moyen de l'élément partiellement déformable 111, comme illustré en figure 11A, ce qui engendre la translation simultanée de l'élément de coupe et de l'élément poussoir.

Alors que l'élément de coupe perfore l'oreille de l'animal et vient en butée sur la collerette de la tête de tube du tube d'échantillonnage, les moyens d'entraînement 442 de l'élément poussoir continuent à pousser l'élément poussoir 23 et l'élément partiellement déformable 111, l'utilisateur poursuivant son action sur le levier 42. Les doigts ou lames ressort 113 s'écartent alors, déclippant les bossages 114 des logements complémentaires prévus dans les moyens d'entraînement 441 de l'élément de coupe, ce qui libère les moyens d'entraînement 441 de l'élément de coupe. Les moyens d'entraînement 441 de l'élément de coupe et les moyens d'entraînement 442 de l'élément poussoir se trouvent alors dans la position de désaccouplement.

Selon une variante, des moyens spécifiques de déformation peuvent être prévus sur le corps de l'outil pour déformer les doigts 113, lorsque l'extrémité libre de ces doigts vient en butée contre ces moyens de déformation.

On note que le ressort primaire 115, prévu entre les moyens d'entraînement 441 de l'élément de coupe et les moyens d'entraînement 442 de l'élément poussoir, permet de maintenir en pression l'élément de coupe contre la collerette de la tête de tube du tube d'échantillonnage, notamment lorsque les doigts ou lames ressort 113 se déforment.

### v) Autre exemple de réalisation

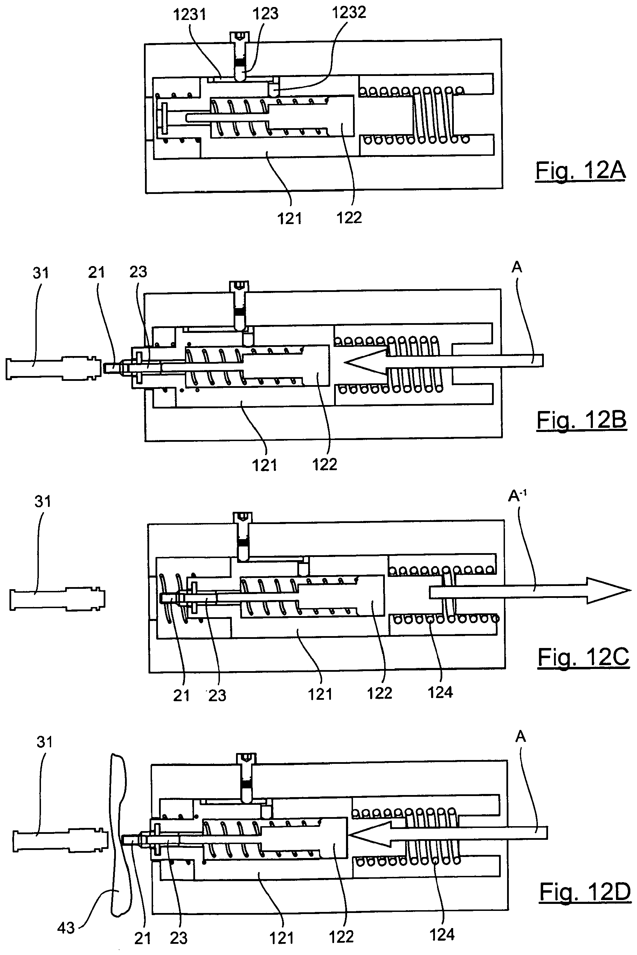

On présente désormais, en relation avec les figures 12A à 12H, un autre exemple de réalisation de l'invention, dit à « inertie ».

Selon cet exemple, les moyens d'entraînement de l'élément de coupe prennent la forme d'un « piston » principal 121, et les moyens d'entraînement de l'élément poussoir la forme d'un « piston » secondaire 122. Les pistons principal et secondaire peuvent coulisser dans le corps de la pince, par exemple dans une première branche, selon un mouvement de translation. Le piston secondaire peut coulisser à l'intérieur du piston principal, également selon un mouvement de translation.

Les moyens d'accouplement comprennent notamment un « doigt de verrouillage » 123, maintenu en position dans le corps de la pince, coopérant avec une fente 1231 prévue dans le piston primaire 121. Une encoche 1232 destinée à libérer le doigt de verrouillage est également prévue à l'une des extrémités de la fente 1231. Lorsque le piston primaire coulisse dans le corps de la pince, la position du doigt de verrouillage 123 dans la fente 1231 est modifiée. Ceci correspond à la position d'accouplement des pistons principal et secondaire. En particulier, lorsque le doigt de verrouillage 123 se trouve en vis-à-vis de l'encoche 1232, il se trouve bloqué en position, libérant ainsi le mouvement du piston secondaire. Ceci correspond à la position de désaccouplement des pistons principal et secondaire.

La figure 12A illustre plus précisément la position des pistons principal 121 et secondaire 122 au repos.

Préalablement à l'opération de prélèvement, les moyens de prélèvement et de stockage sont montés sur le système à pistons. Pour ce faire, comme illustré en figure 12B, l'utilisateur peut avancer le piston principal, et par conséquent le piston secondaire, dont le mouvement est lié au piston principal grâce aux moyens d'accouplement en position d'accouplement, selon la direction de la flèche A. Les moyens de prélèvement comprenant l'élément de coupe 21 (éventuellement solidarisé à un support) et l'élément poussoir 23 peuvent alors être montés sur le piston principal 121. Les moyens de stockage 31 peuvent être montés sur une deuxième branche de la pince. On note que les moyens de prélèvement et de stockage peuvent être maintenus ensemble par l'intermédiaire d'une pièce de liaison. Cet ensemble peut être fixé sur une des branches de la pince, par exemple au niveau des moyens de stockage, par un ensemble de bridage, une bague de verrouillage, etc. Une action de l'utilisateur sur la pince permet de solidariser les moyens de prélèvement sur l'autre branche, par effet de clippage ou de serrage. La pièce de liaison peut par exemple rester sur les moyens de prélèvement, afin de protéger l'élément de coupe préalablement au prélèvement.

Lorsque l'utilisateur est prêt à réaliser le prélèvement, il peut retirer ou éjecter la pièce de liaison, et « armer » la pince en ramenant le système à pistons selon une direction opposée à la flèche A, comme illustré en figure 12C. Cette opération peut être réalisée avec un mécanisme démultipliant l'effort, de façon à compresser un ressort 124 lié au piston principal 121, jusqu'à atteindre une position de verrouillage. Un tel mécanisme prend par exemple la forme d'un écrou avec un pas adapté, d'un système de levier, d'une pompe, d'une crémaillère, d'un mécanisme électrique de type vérin, micromoteur, etc. La compression du ressort 124 permet d'accumuler de l'énergie. Au cours de cette étape, les moyens d'accouplement étant en position d'accouplement, les mouvements des pistons principal et secondaire sont toujours liés.

Selon une variante, non illustrée, l'opération d'armement peut-être effectuée en connectant l'outil à une source d'énergie extérieure (air comprimé en cartouche ou en réseau par exemple).

Comme illustré en figure 12D, l'utilisateur peut alors positionner l'oreille 43 de l'animal entre les deux branches de la pince, et actionner un bouton de déverrouillage, qui libère la compression exercée sur le ressort 124. Le ressort 124 se détend, entraînant un déplacement du piston principal 121 et du piston secondaire 122, toujours en position d'accouplement, en direction de l'oreille de l'animal. Cette action sur un bouton de déverrouillage engendre une accélération importante du piston principal, transformant l'énergie potentielle du ressort 124 en énergie cinétique permettant à l'élément de coupe de perforer l'oreille de l'animal. L'énergie nécessaire au déplacement du piston principal peut ainsi être fournie par la compression/décompression du ressort 124, plutôt que par l'utilisateur.

Selon la variante précédemment mentionnée, l'accélération pourrait être obtenue par la pression de l'air comprimé sur le piston principal, au moyen d'un vérin par exemple.

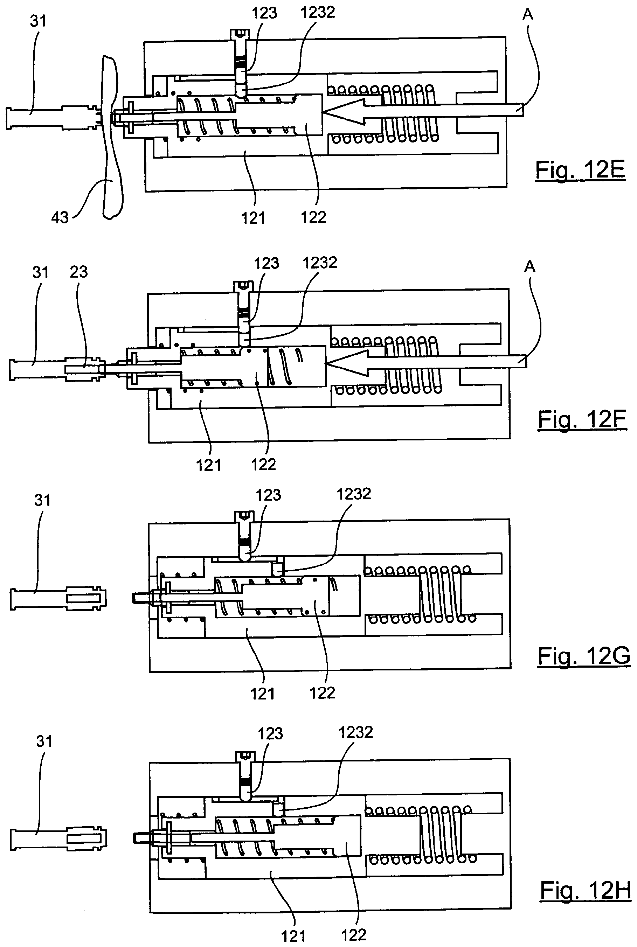

Le piston principal 121 et le piston secondaire 122 continuent leurs mouvements liés jusqu'à ce que l'élément de coupe 21 perfore l'oreille 43 de l'animal, comme illustré en figure 12E. Le mouvement du piston principal 121 s'arrête brusquement lorsque l'élément de coupe 21 vient en butée avec les moyens de prélèvement, notamment la tête de tube 32, et/ou lorsque le doigt de verrouillage 123 pénètre dans l'encoche 1232 prévue à cet effet.

Ceci permet de désaccoupler les moyens d'accouplement, et donc de dissocier les mouvements des pistons principal et secondaire.

En d'autres termes, comme illustré en figure 12F, l'arrêt brutal du mouvement du piston principal 121 lorsque l'élément de coupe 21 vient en butée avec les moyens de prélèvement et/ou lorsque le doigt de verrouillage 123 pénètre dans l'encoche 1232 confère au piston secondaire 122 de l'énergie, suivant la règle de conservation des quantités de mouvement, entraînant un déplacement en translation du piston secondaire 122 propulsant l'élément poussoir en direction de l'oreille de l'animal. L'élément poussoir extrait ainsi l'échantillon découpé de l'élément de coupe, le pousse dans le tube de prélèvement, et vient fermer le tube de prélèvement 31. Selon ce mode de réalisation, le piston secondaire est donc actionné uniquement par inertie.

Le piston secondaire peut notamment présenter une forme spécifique, comme illustrée sur les figures, comprenant un renflement permettant de repousser le doigt de verrouillage 123 hors de l'encoche 1232 pour libérer le mouvement du piston principal. Ainsi, comme illustré en figures 12G et 12H, le piston principal, puis le piston secondaire, peuvent reprendre leur position initiale après libération du piston principal, par exemple sous l'action de ressorts de rappel de faible raideur.

Comme décrit en relation avec les autres modes de réalisation, il est possible pour l'utilisateur d'éjecter l'élément de coupe souillé, par exemple en tirant sur le piston principal selon une direction opposée à la flèche A, une partie du support de l'élément de coupe venant en butée contre un élément prévu à cet effet sur le corps de la pince.

Les autres caractéristiques et avantages décrits précédemment en relation avec les autres modes de réalisation s'appliquent également à ce mode de réalisation, et ne sont donc pas repris en détails ici.

### D) Retrait automatique de l'élément de coupe

Selon un mode de réalisation particulier de l'invention, l'élément de coupe peut reprendre automatiquement sa position initiale, une fois l'échantillon découpé. Ce retrait automatique permet de libérer rapidement l'oreille de l'animal, et évite les risques de coupure avec un élément de coupe souillé, en protégeant l'arête coupante de l'élément de coupe.

Par exemple, ce retrait automatique peut survenir lorsque l'élément de coupe vient en butée sur une surface de butée, comme la tête de tube, ou lorsqu'un doigt de verrouillage pénètre dans une encoche prévue à cet effet selon l'exemple de réalisation décrit précédemment.

Selon un premier exemple, comme décrit ci-dessus en relation avec les figures 12F à 12H, le retrait automatique de l'élément de coupe peut être obtenu en utilisant une forme spécifique pour le piston secondaire, comprenant un renflement permettant de repousser le doigt de verrouillage 123 hors de l'encoche 1232. Ceci permet de libérer le mouvement du piston principal et le ramener en position de repos (ce qui permet de protéger l'arête coupante), et de ramener le piston secondaire en position de repos sous l'action de ressorts de rappel de faible raideur.

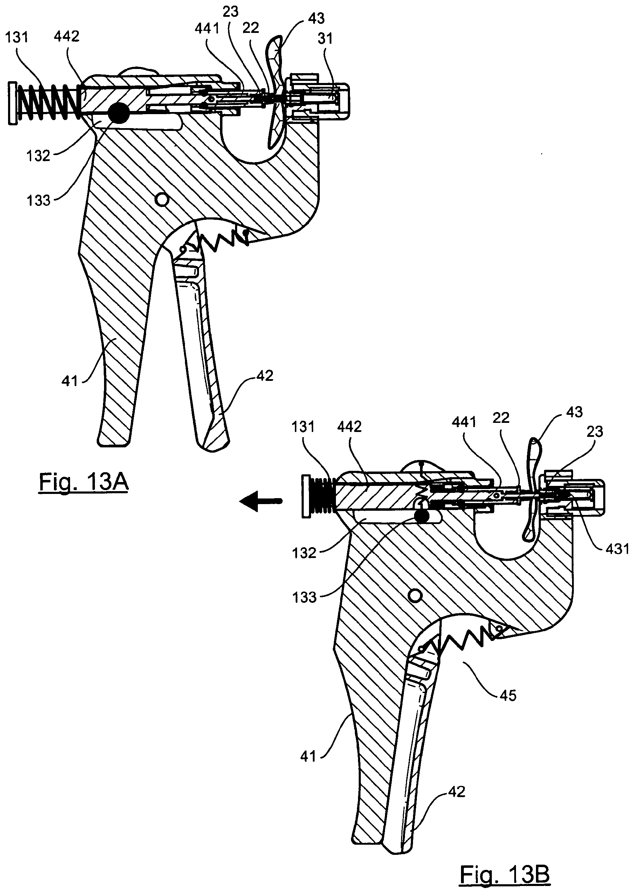

Selon un deuxième exemple, illustré en figures 13A et 13B, le retrait automatique de l'élément de coupe est mis en oeuvre grâce à l'utilisation combinée d'un ressort de rappel 131 lié aux moyens d'entraînement 442 de l'élément poussoir, d'un axe de transmission 133 lié aux moyens d'entraînement 442, et d'une lumière 132 dans le corps 41 de la pince, dans laquelle l'axe de transmission 133 peut se déplacer.

Comme décrit précédemment en relation avec les figures 5A et 5B, l'actionnement du levier 42 engendre tout d'abord une translation simultanée de l'élément de coupe 21 et de l'élément poussoir 23, permettant à l'élément de coupe de découper un échantillon de tissu, jusqu'à venir en butée sur la collerette de la tête de tube du tube d'échantillonnage 31. La poursuite de l'action sur le levier 42 engendre ensuite une translation de l'élément poussoir 23 uniquement, à travers l'élément de coupe 21, engendrant une compression du ressort de rappel 131 contre le corps de la pince.

Le déplacement des moyens d'entraînement 442 de l'élément poussoir entraîne également un déplacement de l'axe de transmission 133 le long de la lumière 132. Selon cette variante, la lumière présente une pente. Ainsi, l'axe de transmission 133 est maintenu dans une encoche prévue à cet effet dans les moyens d'entraînement 442 dans un premier temps, puis au fur et à mesure du déplacement le long de la lumière 132, l'axe de transmission 133 est libéré des moyens d'entraînement 442. Par exemple, l'axe de transmission 133 est maintenu en appui contre la pente de la lumière 132 au moyen d'un ressort 134 solidaire du levier 42.

Une fois l'axe de transmission 133 dégagé des moyens d'entraînement 442, le ressort de rappel 131 se détend et rappelle les moyens d'entraînement de l'élément poussoir 442, puis les moyens d'entraînement de l'élément de coupe 441 (liés par un ressort 135) dans leur position initiale.

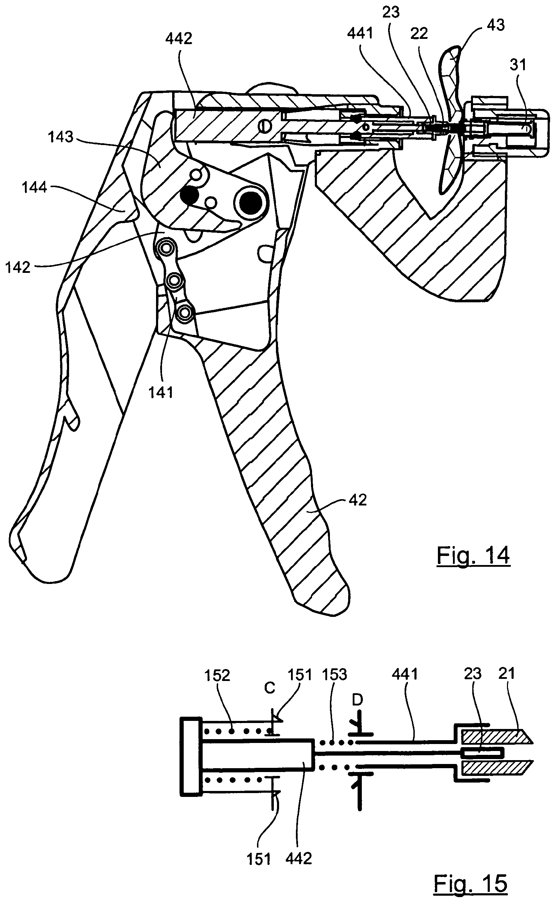

Selon un troisième exemple, illustré en figure 14, le retrait automatique de l'élément de coupe est mis en oeuvre grâce à l'utilisation combinée d'une chaîne 141 formée de deux maillons, d'au moins une flasque 142 et d'une came 143, agissant sur les moyens d'entraînement 442 de l'élément poussoir.

Plus précisément, l'actionnement du levier 42 engendre un mouvement de la chaîne 141, transmettant l'effort nécessaire à la rotation de la flasque 142. La rotation de la flasque 142 actionne la came 143, qui actionne à son tour les moyens d'entraînement de l'élément poussoir et de l'élément de coupe simultanément dans un premier temps, puis de manière dissociée dans un deuxième temps.

En fin de course du levier 42, une butée 144 intégrée au corps de la pince impose à la chaîne 141 de se courber. Elle ne peut alors plus transmettre d'effort à la flasque 142, qui retrouve sa position initiale sous l'effet de ressorts de rappel. Par suite, la came 143 retrouve également sa position initiale. Les moyens d'entraînement de l'élément poussoir 442 et les moyens d'entraînement de l'élément de coupe 441 retrouvent également leur position initiale, puisque aucun effort n'est plus appliqué par la came 143 sur les moyens d'entraînement.

Finalement, la figure 15 illustre un quatrième exemple du retrait automatique de l'élément de coupe, mettant en oeuvre un mécanisme à base de crochets.

Selon cet exemple, les moyens d'entraînement de l'élément poussoir 442 sont munis de crochets 151, utilisés pour armer la pince et pour le retrait automatique de l'élément de coupe une fois l'opération de prélèvement terminée.

L'armement de la pince s'effectue par exemple en poussant les moyens d'entraînement de l'élément poussoir 442 jusqu'à ce que les crochets 151 viennent crocheter un élément C correspondant de la pince, entraînant la compression d'un ressort de rappel 152 entre la base des moyens d'entraînement 442 et l'élément crocheté C.

Le déplacement de l'élément crocheté C et des moyens d'entraînement 442de l'élément de poussoir, par actionnement de la pince par exemple, engendre tout d'abord une translation simultanée de l'élément de coupe 21 et de l'élément poussoir 23 (les moyens d'entraînement 441 de l'élément de coupe étant liés aux moyens d'entraînement 442 de l'élément de poussoir par l'intermédiaire d'un ressort 153). Lorsque l'élément de coupe arrive en butée sur la collerette de la tête de tube du tube d'échantillonnage, le ressort 153 se compresse et seul l'élément poussoir 23, poussé par les moyens d'entraînement 442, poursuit sa course à travers l'élément de coupe 21.

La compression du ressort 153 amène l'élément crocheté C en contact avec un élément correspondant de « décrochetage » D, permettant de libérer les crochets 151 l'élément crocheté C. Une fois les crochets 151 des moyens d'entraînement 442 déverrouillés, le ressort de rappel 152 se détend, et rappelle les moyens d'entraînement 442de l'élément poussoir, puis les moyens d'entraînement 441 de l'élément de coupe (par l'intermédiaire du ressort 153) dans leur position initiale.

Il est à noter que ces quatre exemples de mise en oeuvre sont décrits dans le cas où la pince fonctionne avec des moyens de prélèvement comprenant à la fois un élément de coupe et un élément poussoir. Ces mécanismes pourraient bien sûr être mis en oeuvre dans une pince « classique », fonctionnant avec des moyens de prélèvement comprenant un simple emporte-pièce, non combiné avec un élément poussoir.

### E) Variantes

Quelque soit le mode de réalisation envisagé, l'outil de prélèvement selon l'invention confère un mouvement de translation aux éléments de coupe et poussoir selon une même direction, les deux mouvements étant liés dans un premier temps et déliés dans un deuxième temps.

On rappelle que ces deux temps correspondent à une seule action de l'utilisateur sur l'outil de prélèvement, le premier temps correspondant à une première portion de la course du levier entre une position initiale et une position intermédiaire, définissant une position d'accouplement, et le deuxième temps correspondant à une deuxième portion de la course du levier entre la position intermédiaire et une position finale, définissant une position de désaccouplement.

D'autres moyens d'accouplement peuvent également être envisagés, du moment qu'ils rentrent dans le cadre de la présente invention telle que définie par les revendications. Dans le mode de réalisation décrit, l'outil de prélèvement est actionné manuellement. Bien entendu, il pourrait être actionné au moyen d'une énergie électrique, pneumatique, ou autre.

De plus, dans le mode de réalisation décrit, le levier est mobile en rotation entre les positions initiale, intermédiaire et finale. Selon une variante non illustrée, le levier peut être mobile en translation entre ces différentes positions.

Selon encore un autre mode de réalisation, l'outil de prélèvement peut apposer une marque d'identification (visuelle et/ou électronique), simultanément au prélèvement de l'échantillon. Avantageusement, cette marque d'identification porte un identifiant lié à un identifiant des moyens de prélèvement et/ou de stockage.

## Revendications

1. Outil de prélèvement d'un échantillon de tissu animal,
destiné à coopérer avec des moyens de prélèvement comprenant au moins un élément de coupe (21) apte à découper ledit échantillon et un élément poussoir (23) mobile par rapport audit élément de coupe, apte à pousser ledit échantillon dans des moyens de stockage après découpe, lesdits moyens de stockage comprenant un tube d'échantillonnage (31) et une tête de tube (32), ledit outil comprenant :
- des moyens d'entraînement (441) dudit élément de coupe (21), permettant de mouvoir ledit élément de coupe selon un axe de translation, en direction dudit tissu,
- des moyens d'entraînement (442) dudit élément poussoir (23), permettant de mouvoir ledit élément poussoir selon ledit axe de translation en direction dudit tissu, jusqu'à insertion d'au moins une portion dudit élément poussoir dans ladite tête de tube,
- des moyens d'accouplement réversible (91, 92) desdits moyens d'entraînement de l'élément de coupe et desdits moyens d'entraînement de l'élément poussoir, lesdits moyens d'accouplement étant mobiles entre deux positions :
∘ une position d'accouplement permettant de lier les mouvements desdits éléments de coupe et poussoir, et
∘ une position de désaccouplement permettant de dissocier les mouvements desdits éléments de coupe et poussoir.
lesdits moyens d'accouplement mettant en oeuvre au moins un élément de couplage mobile (92), apte à coopérer avec un logement (91) ménagé à cet effet dans lesdits moyens d'entraînement dudit élément de coupe, le ou lesdits éléments de couplage étant maintenus dans ledit logement dans ladite position d'accouplement et libérés dans ladite position de dés accouplement.

2. Outil de prélèvement selon la revendication 1, **caractérisé en ce qu'**il comprend au moins une poignée articulée formant levier (42), mobile sur une course prédéterminée comprenant une première portion et une deuxième portion,
ledit levier comprenant des moyens d'action sur lesdits moyens d'accouplement permettant le passage de ladite position d'accouplement sur ladite première portion à ladite position de désaccouplement sur ladite deuxième portion.

3. Outil de prélèvement selon l'une quelconque des revendications 1 et 2, **caractérisé en ce que** lesdits moyens d'entraînement de l'élément poussoir comprennent au moins une gorge (93), ledit logement étant en regard de ladite gorge dans ladite position d'accouplement.

4. Outil de prélèvement selon l'une quelconque des revendications 1 à 3, **caractérisé en ce que** ledit au moins un élément de couplage est une bille (92).

5. Outil de prélèvement selon la revendication 4, **caractérisé en ce que** ledit logement est une lumière traversante et **en ce que** ladite bille est maintenue dans ledit logement par l'intermédiaire d'une pièce coulisseau (94),
ladite pièce coulisseau étant conformée pour définir des moyens de maintien maintenant ladite bille dans ladite gorge dans ladite position d'accouplement, et des moyens de libération libérant ladite bille de ladite gorge dans ladite position de désaccouplement.

6. Outil de prélèvement selon la revendication 5, **caractérisé en ce que** ladite pièce coulisseau présente une forme de révolution autour dudit axe de translation, définissant un manchon comprenant au moins deux sections (941, 942) de diamètres distincts, lesdits moyens de maintien correspondant à la section (941) de diamètre inférieur et lesdits moyens de libération à la section (942) de diamètre supérieur.

7. Outil de prélèvement selon l'une quelconque des revendications 5 et 6, **caractérisé en ce que** ladite pièce coulisseau est reliée auxdits moyens d'entraînement de l'élément poussoir par un ressort (95), dit ressort coulisseau, ledit ressort coulisseau étant dans un état de repos dans ladite position d'accouplement et dans un état de compression dans ladite position de désaccouplement.

8. Outil de prélèvement selon l'une quelconque des revendications 1 à 7, **caractérisé en ce qu'**il comprend des moyens de verrouillage desdits moyens de stockage sur une branche dudit outil.

9. Outil de prélèvement selon l'une quelconque des revendications 1 à 8 **caractérisé en ce qu'**il comprend des moyens d'éjection dudit élément de coupe.

10. Outil de prélèvement selon l'une quelconque des revendications 1 à 9, **caractérisé en ce qu'**il comprend des moyens de retrait automatique dudit élément de coupe.

## Patentansprüche

1. Werkzeug zur Entnahme einer Probe aus Tiergewebe,
das dazu bestimmt ist, mit Entnahmemitteln zusammenzuwirken, die zumindest ein Schneideelement (21), das dazu geeignet ist, die Probe herauszuschneiden, und ein gegenüber dem Schneideelement bewegbares Schiebeelement (23), das dazu geeignet ist, die Probe nach dem Herausschneiden in Lagermittel zu schieben, enthalten,
wobei die Lagermittel eine Probentube (31) und einen Tubenkopf (32) enthalten, wobei das Werkzeug Folgendes enthält:
- Antriebsmittel (441) des Schneideelements (21), welche ermöglichen, das Schneideelement entlang einer Translationsachse in Richtung des Gewebes zu bewegen,
- Antriebsmittel (442) des Schiebeelements (23), welche ermöglichen, das Schiebeelement entlang der Translationsachse bis zum Einführen von zumindest einem Teil des Schiebeelements in den Tubenkopf in Richtung des Gewebes zu bewegen,
- Lösbare Kopplungsmittel (91, 92) der Antriebsmittel des Schneideelements und der Antriebsmittel des Schiebeelements,
wobei die Kopplungsmittel zwischen folgenden zwei Positionen bewegbar sind:
o einer Kopplungsposition, die es ermöglicht, die Bewegungen des Schneideelements und des Schiebeelements zu verbinden, und
o eine Entkopplungsposition, die es ermöglicht, die Bewegungen des Schneideelements und des Schiebeelements voneinander zu trennen,
wobei die Kopplungsmittel zumindest ein bewegbares Kopplungselement (92) umsetzen, das dazu geeignet ist, mit einer Aufnahme (91) zusammenzuwirken, welche zu diesem Zweck in den Antriebsmitteln des Schneideelements vorgesehen ist,
wobei das oder die Kopplungselemente in der Kopplungsposition in der Aufnahme gehalten wird bzw. werden und in der Entkopplungsposition freigegeben wird bzw. werden.

2. Werkzeug zur Entnahme nach Anspruch 1, **dadurch gekennzeichnet, dass** es zumindest einen gelenkigen Griff enthält, der einen Hebel (42) ausbildet, der entlang eines vorbestimmten Weges, der einen ersten Teil und einen zweiten Teil enthält, beweglich ist, wobei der Hebel Mittel zur Betätigung der Kopplungsmittel enthält, die den Übergang von der Kopplungsposition an dem ersten Teil auf die Entkopplungsposition an dem zweiten Teil ermöglichen.

3. Werkzeug zur Entnahme nach einem beliebigen der Ansprüche 1 und 2, **dadurch gekennzeichnet, dass** die Antriebsmittel des Schiebeelements zumindest eine Nut (93) enthalten, wobei die Aufnahme in der Kopplungsposition der Nut gegenüber ist.

4. Werkzeug zur Entnahme nach einem beliebigen der Ansprüche 1 bis 3, **dadurch gekennzeichnet, dass** das wenigstens eine Kopplungselement eine Kugel (92) ist.

5. Werkzeug zur Entnahme nach Anspruch 4, **dadurch gekennzeichnet, dass** die Aufnahme ein Durchgangsloch ist und dadurch, dass die Kugel in der Aufnahme mittels eines Schiebestücks (94) gehalten wird, wobei das Schiebestück angepasst ist, um Haltemittel, die die Kugel in der Kopplungsposition in der Nut halten, und Freigabemittel, die die Kugel in der Entkopplungsposition aus der Nut freigeben, festzulegen.

6. Werkzeug zur Entnahme nach Anspruch 5, **dadurch gekennzeichnet, dass** das Schiebestück eine rotationssymmetrische Form um die Translationsachse aufweist, die eine Muffe festlegt, die zumindest zwei Abschnitte (941, 942) von verschiedenem Durchmesser enthält, wobei die Haltemittel dem Abschnitt (941) mit geringerem Durchmesser und die Freigabemittel dem Abschnitt (942) mit größerem Durchmesser entsprechen.

7. Werkzeug zur Entnahme nach einem beliebigen der Ansprüche 5 und 6, **dadurch gekennzeichnet, dass** das Schiebestück mit den Antriebsmitteln des Schiebeelements durch eine Feder (95) in Verbindung steht, genannt Schiebefeder, wobei die Schiebefeder in der Kopplungsposition in einem Zustand der Entspannung und in der Entkopplungsposition in einem Zustand der Komprimierung ist.

8. Werkzeug zur Entnahme nach einem beliebigen der Ansprüche 1 bis 7, **dadurch gekennzeichnet, dass** es Verriegelungsmittel der Lagermittel auf einem Schenkel des Werkzeugs enthält.

9. Werkzeug zur Entnahme nach einem beliebigen der Ansprüche 1 bis 8, **dadurch gekennzeichnet, dass** es Ausstoßmittel des Schneideelements enthält.

10. Werkzeug zur Entnahme nach einem beliebigen der Ansprüche 1 bis 9, **dadurch gekennzeichnet, dass** es Mittel zur automatischen Rückführung des Schneideelements enthält.

## Claims

1. Tool for collecting a sample of animal tissue,
intended for cooperating with collecting means comprising at least one cutting element (21) designed to cut out said sample and one pusher element (23) that is mobile relatively to said cutting element, designed to push said sample into storage means after cutting, said storage means comprising a sampling tube (31) and a tube head (32),
said tool comprising :
- means (441) for driving said cutting element (21), enabling said cutting element to be moved along an axis of translation, in the direction of said tissue,
- means (442) for driving said pusher element (23), enabling said pusher element to be moved along said axis of translation in the direction of said tissue until at least one portion of said pusher element is inserted into said tube head,
- means for the reversible coupling (91, 92) of said means for driving the cutting element and said means for driving the pusher element, said coupling means being mobile between two positions:
∘ a coupling position enabling the movements of said cutting and pusher elements to be linked together, and
∘ a decoupling position enabling the movements of the cutting and pusher elements to be dissociated,
said coupling means implementing at least one mobile coupling element (92) intended for cooperating with a housing (91) designed for this purpose in said means for driving said cutting element, said coupling element or elements being held in said housing in said coupling position and being released in said decoupling position.

2. Tool for collecting according to claim 1, **characterized in that** it comprises at least one hinged grip forming a lever (42) that is mobile on a predetermined course comprising a first portion and a second portion,
said lever comprising means of action on said coupling means enabling passage from said coupling position on said first portion to said decoupling position on said second portion.

3. Tool for collecting according to any one of the claims 1 or 2, **characterized in that** said means for driving the pusher element comprise at least one groove (93), said housing facing said groove in said coupling position.

4. Tool for collecting according to any one of the claims 1 to 3, **characterized in that** said at least one coupling element is a ball (92).

5. Tool for collecting according to claim 4, **characterized in that** said housing is a through aperture and **in that** said ball is held in said housing by means of a sliding part (94),
said sliding part being shaped to define holding means holding said ball in said groove in said coupling position, and releasing means releasing said ball from said groove in said decoupling position.

6. Tool for collecting according to claim 5, **characterized in that** said sliding part has a shape generated by revolution about said axis of translation defining a sleeve comprising at least two sections (941, 942) of distinct diameters, said holding means corresponding to the section (941) of smaller diameter and said releasing means corresponding to the section (942) of greater diameter.

7. Tool for collecting according to any one of the claims 5 and 6, **characterized in that** said sliding part is connected to said means for driving the pusher element by a spring (95), called a slide spring, said slide spring being in an idle state in said coupling position and in a compressed state in said decoupling position.

8. Tool for collecting according to any one of the claims 1 to 7, **characterized in that** it comprises means for locking said storage means on to one arm of said tool.

9. Tool for collecting according to any one of the claims 1 to 8 **characterized in that** it comprises means for ejecting said cutting element.

10. Tool for collecting according to any one of the claims 1 to 9, **characterized in that** it comprises means for the automatic withdrawal of said cutting element.
